# EUROPEAN PATENT APPLICATION

(11) **EP 3 048 578 A1**
(43) Date of publication of application: **27.07.2016**
(21) Application number: 14845546.2
(22) Date of filing: 11.09.2014
(51) Int. Cl.: G06Q 50/22, G06Q 10/06

(54) **SERVER FOR HOME-VISIT-SERVICE MANAGEMENT ASSISTANCE SYSTEM, CONTROL METHOD FOR SAID SERVER, AND CONTROL PROGRAM FOR SAID SERVER**

(30) Priority: 18.09.2013 JP 2013192838
(71) Applicant: Nagai, Chieko, Sakaide-shi, Kagawa 762-0025 (JP)
(72) Inventor: NAGAI,Chieko, Kagawa 7620025 (JP); SUGIMOTO, Yukio, Tokyo 1048280 (JP)
(74) Representative: Delumeau, François Guy
(86) International application number: PCT/JP2014/074114
(87) International publication number: WO 2015/041147

(57) **Abstract**

The purpose of the present invention is to provide a server or the like for a home-visit-service management assistance system, said server enabling the utilization opportunities of a home-visit service to be improved. A server (3) according to the present invention is provided with: a storage unit (32) having, stored therein, information related to preferred dates and times of a first service provider, information related to preferred dates and times of a second service provider, and information related to preferred dates and times of a service user; a service-provision-plan information generation unit (331) which allocates, to each of the dates and times indicated by the information related to the preferred dates and times of the service user, each of the dates and times indicated by the information related to the preferred dates and times of the second service provider, further allocates each of the dates and times indicated by the information related to the preferred dates and times of the first service provider, to each of the unallocated dates and times among each of the dates and times indicated by the information related to the preferred dates and times of the service user, said unallocated dates and times being those not having, allocated thereto, any of the dates and times indicated by the information related to the preferred dates and times of the second service provider, and generates service-provision-plan information on the basis of the allocation results; and a service-provision-plan information reference unit (332) for transmitting the service-provision-plan information.

## Description

### FIELD

The present invention relates to a server in a visiting service management support system, a control method thereof, and a control program thereof.

### BACKGROUND

The average life span of the Japanese has lengthened and the ratio of the aged to the population is also increasing. As the population in need of care is also increasing because of aging of the society, the Public Nursing Care Insurance Law (also referred to as the Long-Term Care Insurance Act (Law)) was put in force in April 2000, and the entry of the private sector into the care service-related business has expanded.

As described above, although the number of care service providers has increased as result of the enforcement of the Public Nursing Care Insurance Law, there are a variety of problems with regard to the business operating environment. Although an attempt to improve the system was made by the revised Public Nursing Care Insurance Law in 2006 and 2012, both the service users (e.g., persons requiring care, persons requiring assistance, etc.) and the service providers (e.g., nurses, care workers, care attendants, etc.) still feel discontented.

Specifcally, there are the following problems.
(1) It is necessary to exchange a large number of documents among a person who desires to use a service by the time to use a care service, a care manager, a service provider, etc. A care manager is a person who receives a request for advice from a service user, clarifies problems and objectives when the service user uses the Public Nursing Care Insurance services, creates a plan (care plan) that summarizes the contents and schedules of the services that the service user uses, and establishes contact and arranges adjustment with the service provider whose service the service user needs.
(2) When the service provision is started actually, for the reports of medical treatment and care, a large number of documents need to be prepared, recorded, and presented, and therefore nearly half the time required to deal with one visit is spent by the clerical work other than the actual service.
(3) Further, these documents are also confidential, and therefore they are forced to be basically carried by hand-holding, and much time is required to move from the visit to back to the office.

Thus, the cost of the care service business as a whole rises, resulting in an increase in the burden of the service user. Further, the total hours spent at work are long, and therefore even the qualified service provider of caliber will lose the chance to work. In other words, since the total hours spent at work are long, the number of full-time staff tends to run short. Even if an attempt to cover the shortage by part-time staff, the time restriction of the part-time staff is severe, and therefore it is difficult to sufficiently cover the shortage. Further, although there are providers that perform part of the clerical work in place of the staff in order to reduce the burden of the clerical work, this will lead to the cost of outsourcing, and therefore it is not possible for a small-scale care service provider to resolve the problem of a reduction in burden on the management side. Thus, despite the fact that the demand for the care service has expanded, the number of staff actually engaged in the service provision is insufficient and the care service provider spends much time creating a visiting schedule of the service provider. Further, there has occurred a state where it is not possible for a small-scale care service provider to efficiently provide services. The state such as above has similarly occurred in the visiting nursing service or the like, not limited to the visiting care service.

A variety of solutions have been proposed for the problem such as this. For example, Patent Document 1 has disclosed matching between a person who desires to use a service and a service provider. In particular, matching has been disclosed in which the desired conditions of both are posted up on a dedicated bulletin board and those whose conditions match with each other are matched and managed on the system.

### Related Document

[Patent Document 1] Japanese Laid Open Patent No. 2002-074058

### SUMMARY

However, even with the solution such as this, it is not possible to resolve the problem to improve the chance to work of the qualified service provider of caliber as described above.

The present invention has been made in view of the circumstance such as this, and an object of the present invention is to provide a server in a visiting service management support system that may improve the chance to work of the service provider by devising scheduling of the service provider, as well as improving the service provision efficiency by reducing the burden of task of the service provider, the person in charge of clerical work, the manager, etc., to secure flexibility in scheduling when the care service provider provides services, and thereby improving the chance to use a service of the service user, a control method of the server, and a control program of the server.

A server according to the present invention is a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including: a communication unit configured to communicate with the first terminal and the second terminal, respectively; a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use; a service scheduling information creation unit configured to assign each date and time indicated by the second service provider's desired date and time information to each date and time indicated by the service user's desired date and time information, to further assign each date and time indicated by the first service provider's desired date and time information to each date and time to which each date and time indicated by the second service provider's desired date and time information is not assigned among each date and time indicated by the service user's desired date and time information, to create service scheduling information including a service user, a first service provider or a second service provider, and a service provision date and time based on the assignment results, and to store the service scheduling information in the storage unit; and a service scheduling information reference unit configured to transmit the service scheduling information to the first terminal or the second terminal via the communication unit.

A server according to the present invention is a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including: a communication unit configured to communicate with the first terminal and the second terminal, respectively; a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use; a service scheduling information creation unit configured to simultaneously assign each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information, to create service scheduling information including a service user, a first service provider or a second service provider, and a service provision date and time based on the assignment results, and to store the service scheduling information in the storage unit; and a service scheduling information reference unit configured to transmit the service scheduling information to the first terminal or the second terminal via the communication unit.

In the server according to the present invention, if,the assignment results are modified, it is preferable for the service scheduling information creation unit to perform the assignment processing again so that the modification is reflected in the assignment results.

In the server according to the present invention, it is preferable for the service scheduling information creation unit to perform the assignment processing based on the service user's desired date and time information relating to a service user belonging to a predetermined group of a plurality of groups, and the first service provider's desired date and time information relating to a first service provider belonging to a predetermined group of a plurality of groups having a system different from that of the plurality of groups and the second service provider's desired date and time information relating to a second service provider.

In the server according to the present invention, it is preferable for the storage unit to further store service user management information including service user attribute information and a service provision record relating to the service user for each service user, and for the server to further include a service user management information reference unit configured to transmit the service user management information to the first terminal or the second terminal via the communication unit.

In the server according to the present invention, it is preferable to further include a service user management information updating unit configured to transmit a service provision record input form relating to a service user to the first terminal or the second terminal via the communication unit and to store the service provision record received from the first terminal or the second terminal via the communication unit in the storage unit as a service provision record relating to the service user.

A control method of a server according to the present invention is a control method of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control method including, by the server: assigning each date and time indicated by the second service provider's desired date and time information to each date and time indicated by the service user's desired date and time information; assigning each date and time indicated by the first service provider's desired date and time information to each date and time to which each date and time indicated by the second service provider's desired date and time information is not assigned among each date and time indicated by the service user's desired date and time information; creating service scheduling information including a service user, a first service provider or a second service provider, and a service provision date and time based on the assignment results; storing the service scheduling information in the storage unit; and transmitting the service scheduling information to the first terminal or the second terminal.

A control method of a server according to the present invention is a control method of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control method including, by the server: simultaneously assigning each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information; creating service scheduling information including a service user, a first service provider or a second service provider, and a service provision date and time based on the assignment results; storing the service scheduling information in the storage unit; and transmitting the service scheduling information to the first terminal or the second terminal.

A control program of a server according to the present invention is a control program of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control program causing the server to; assign each date and time indicated by the second service provider's desired date and time information to each date and time indicated by the service user's desired date and time information; assign each date and time indicated by the first service provider's desired date and time information to each date and time to which each date and time indicated by the second service provider's desired date and time information is not assigned among each date and time indicated by the service user's desired date and time information; create service scheduling information including a service user, a first service provider or a second service provider, and a service provision date and time based on the assignment results; store the service scheduling information in the storage unit; and transmit the service scheduling information to the first terminal or the second terminal.

A control program of a server according to the present invention is a control program of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control program causing the server to: simultaneously assign each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information; create service scheduling information including a service user, a first service provider or a second service provider, and a service provision date and time based on the assignment results; store the service scheduling information in the storage unit; and transmit the service scheduling information to the first terminal or the second terminal.

The server, the control method thereof, and the control program thereof may improve the chance to work of the service provider, and thereby, improving the chance to use a service of the service user by preferentially assigning the part-time staff over the full-time staff in scheduling the service provider, since it is easier for the part-time staff to perform the service provision task in the time period desired by the part-time staff him/herself.

### BRIEF DESCRIPTION OF THE DRAWINGS

FIG. 1 is a diagram illustrating an example of an outline configuration of a visiting service management support system;
FIG. 2 is a diagram illustrating an example of an outline configuration of a mobile terminal;
FIG. 3 is a diagram illustrating an example of an outline configuration of a server;
FIGs. 4A to 4F are each a diagram illustrating an example of a data structure of each of various management tables;
FIGs. 5A to 5C are each a diagram illustrating another example of a data structure of each of various management tables;
FIGs. 6A to 6D are each a diagram illustrating an example of a display screen of the mobile terminal;
FIGs. 7A to 7D are each a diagram illustrating another example of a display screen of the mobile terminal;
FIG. 8 is a diagram illustrating an example of an operation flow of the server;
FIGs. 9A and 9B are each a diagram illustrating another example of the operation flow of the server; and
FIGs. 10A and 10B are each a diagram illustrating still another example of the operation flow of the server.

### DESCRIPTION OF EMBODIMENTS

Hereinafter, with reference to the drawings, a variety of embodiments of the present invention are explained. However, it should be noted that the technical scope of the present invention is not limited to those embodiments, but encompasses the inventions described in the claims and equivalents thereof.

### (1) Outline of the present embodiment

In the present embodiment, a manager (including a service provider) creates service scheduling indicating which service provider provides which service user with which service. It is assumed that the service provider includes a full-time staff who provides a service in a working pattern in which the full-time staff works for a long time and continuously and a part-time staff who provides a service in a working pattern in which the part-time staff works for a shorter time and less continuously than the full-time staff, and the manager creates the service scheduling by giving priority to the part-time staff over the full-time staff. The service provider provides a service by appropriately referring to the service scheduling. Then, after providing a service, the service provider creates a service provision record.

In the present embodiment, although the visiting care service is supposed as the service provided by the service provider, the present invention is not limited to this. The service only needs to be one to which the present invention can be applied, and for example, the service may be the visiting nursing service or the visiting medical treatment service, or the care service, the nursing service, the medical treatment service, etc., in facilities, such as hospitals. The visiting medical treatment service is the service provided by a doctor periodically visiting a service user's home and performing medical treatment, remedy, and prescription of drug, receiving a request for advice, giving instructions, etc.

The manager or the service provider gives a mobile terminal instructions to create or refer to service scheduling, to create a service provision record, etc., (hereinafter, referred to as "creation of service scheduling or the like"). The mobile terminal makes a request to the server for creation of service scheduling or the like in accordance with instructions from a user. In response to the request from the mobile terminal, the server performs creation of service scheduling or the like.

### (2) Configuration of visiting service management support system 1

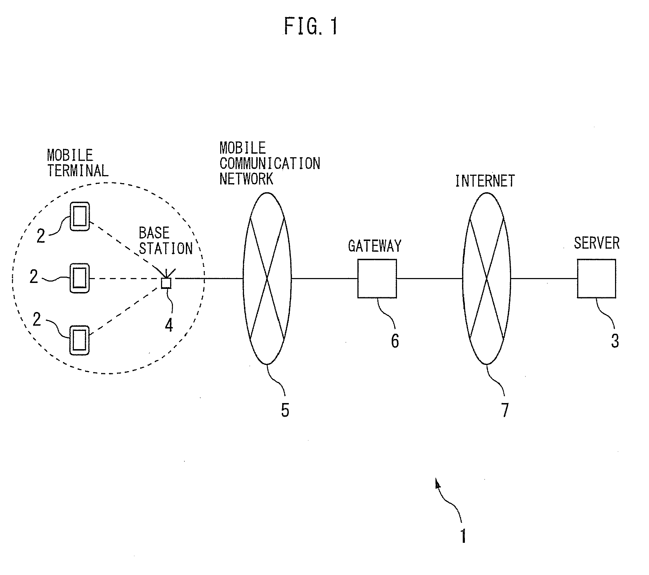

FIG. 1 is a diagram illustrating an example of an outline configuration of a visiting service management support system 1.

The visiting service management support system 1 includes at least one mobile terminal 2 and a server 3. The mobile terminal 2 and the server 3 are connected to each other via a communication network, for example, via a base station 4, a mobile communication network 5, a gateway 6, and the Internet 7. A program (e.g., a browsing program) that is executed in the mobile terminal 2 and a program (e.g., a visiting service management support program) that is executed in the server 3 perform communication by using a communication protocol, such as the hypertext transfer protocol (HTTP).

### (2.1) Configuration of mobile terminal 2

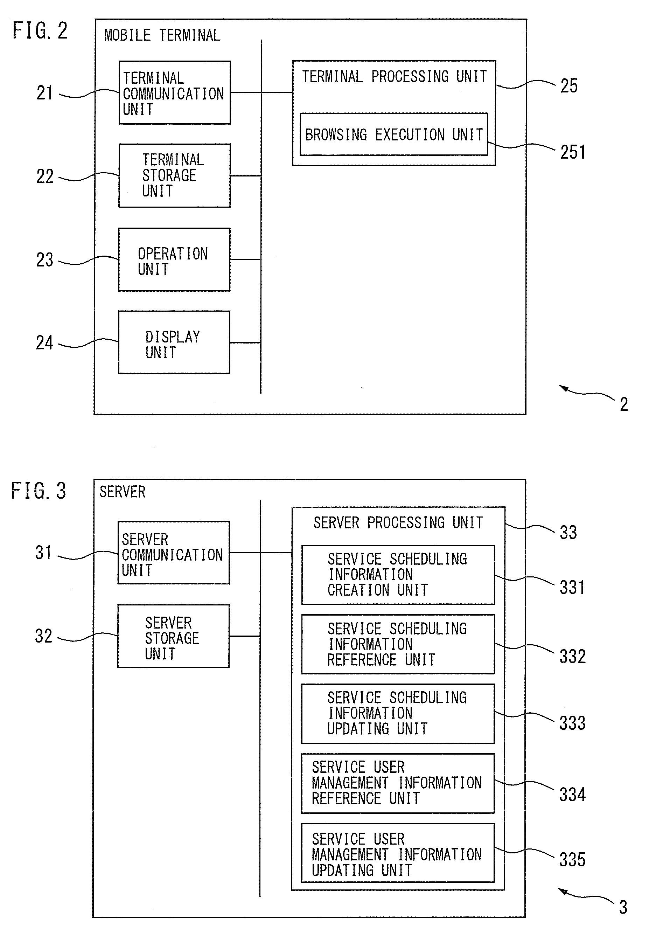

FIG. 2 is a diagram illustrating an example of an outline configuration of the mobile terminal 2.

The mobile terminal 2 connects to the server 3 via the base station 4, the mobile communication network 5, the gateway 6, and the Internet 7 and communicates with the server 3. The mobile terminal 2 makes a request to the server 3 for creation of service scheduling or the like in accordance with an operation by a user through an operation unit 23 (button or the like). Further, the mobile terminal 2 receives display data relating to creation of service scheduling or the like from the server 3 and displays the data. To this end, the mobile terminal 2 includes a terminal communication unit 21, a terminal storage unit 22, the operation unit 23, a display unit 24, and a terminal processing unit 25.

In the present embodiment, although a tablet terminal is supposed as the mobile terminal 2, the present invention is not limited to this. The mobile terminal 2 only needs to be one to which the present invention can be applied and, for example, the mobile terminal 2 may be a tablet PC, a note PC, a multifunction mobile telephone (so-called "smart phone"), a mobile telephone (so-called "feature phone"), a personal digital assistant (PDA), etc.

The terminal communication unit 21 includes a communication interface circuit including an antenna whose reception band is a predetermined frequency band, and connects the mobile terminal 2 to a wireless communication network. The terminal communication unit 21 establishes a wireless signal circuit line by the CDMA (Code Division Multiple Access) system or the like with the base station 4 via a channel assigned by the base station 4 and performs communication with the base station 4. Then, the terminal communication unit 21 transmits data supplied from the terminal processing unit 25 to the server 3 or the like. Further, the terminal communication unit 21 supplies data received from the server 3 or the like to the terminal processing unit 25.

The terminal storage unit 22 includes at least one of, for example, a semiconductor memory device, a magnetic disk device, and an optical disk device. The terminal storage unit 22 stores an operating system program, a driver program, an application program, data, etc., which are used in the processing by the terminal processing unit 25. For example, the terminal storage unit 22 stores an input device driver program that controls the operation unit 23, an output device driver program that controls the display unit 24, etc., as the driver programs. Further, the terminal storage unit 22 stores a browsing program or the like that acquires and displays display data relating to creation of service scheduling or the like as the application program. Furthermore, the terminal storage unit 22 stores display data, video data, image data, etc., relating to creation of service scheduling or the like as the data. Furthermore, the terminal storage unit 22 may temporarily store data relating to predetermined processing.

The operation unit 23 may be any device as long as the device is capable of operating the mobile terminal 2 and is, for example, a touch pad, a keyboard, etc. A user may input characters, figures, etc., by using the operation unit 23. When operated by a user, the operation unit 23 generates a signal corresponding to the operation. Then, the generated signal is supplied to the terminal processing unit 25 as user's instructions.

The display unit 24 may also be any device as long as the device is capable of displaying videos, images, etc., and is, for example, a liquid crystal display, an organic EL (Electro-Luminescence) display, etc. The display unit 24 displays a video in accordance with video data supplied from the terminal processing unit 25, an image in accordance with image data or the like.

The terminal processing unit 25 includes one or a plurality of processors and peripheral circuits thereof. The terminal processing unit 25 centralizedly controls the entire operation of the mobile terminal 2 and is, for example, a CPU (Central Processing Unit). The terminal processing unit 25 controls the operation of the terminal communication unit 21, the display unit 24, etc., so that various kinds of processing of the mobile terminal 2 are performed by an appropriate procedure in accordance with programs stored in the terminal storage unit 22, the operations of the operation unit 23, etc. The terminal processing unit 25 performs processing based on the programs (operating system program, driver program, application program, etc.) stored in the terminal storage unit 22. Further, the terminal processing unit 25 may execute a plurality of programs (application program or the like) in parallel.

### (2.1.1) Configuration of terminal processing unit 25

The terminal processing unit 25 includes at least a browsing execution unit 251. Each of these units is a function module that is implemented by a program executed by a processor included in the terminal processing unit 25. Alternatively, each of these units may be mounted on the mobile terminal 2 as firmware.

The browsing execution unit 251 acquires and displays display data relating to creation of service scheduling or the like. In other words, the browsing execution unit 251 transmits a request to acquire display data relating to creation of service scheduling or the like to the server 3 via the terminal communication unit 21 in accordance with instructions from a user. Further, the browsing execution unit 251 receives corresponding display data from the server 3 via the terminal communication unit 21. The browsing execution unit 251 creates drawing data based on the received display data. In other words, the browsing execution unit 251 specifies control data and contents data by analyzing the received display data and creates drawing data by laying out the specified contents data in accordance with the similarly specified control data. Then, the browsing execution unit 251 outputs the created drawing data to the display unit 24.

### (2.2) Configuration of server 3

FIG. 3 is a diagram illustrating an example of an outline configuration of the server 3.

The server 3 performs creation of service scheduling or the like in accordance with a request from the mobile terminal 2. Further, the server 3 creates display data relating to creation of service scheduling or the like and transmits the display data to the mobile terminal 2. To this end, the server 3 includes a server communication unit 31, a server storage unit 32, and a server processing unit 33.

The server communication unit 31 includes a communication interface circuit for connecting the server 3 to the Internet 7 and performs communication with the Internet 7. Then, the server communication unit 31 supplies data received from the mobile terminal 2 or the like to the server processing unit 33. Further, the server communication unit 31 transmits data supplied from the server processing unit 33 to the mobile terminal 2 or the like.

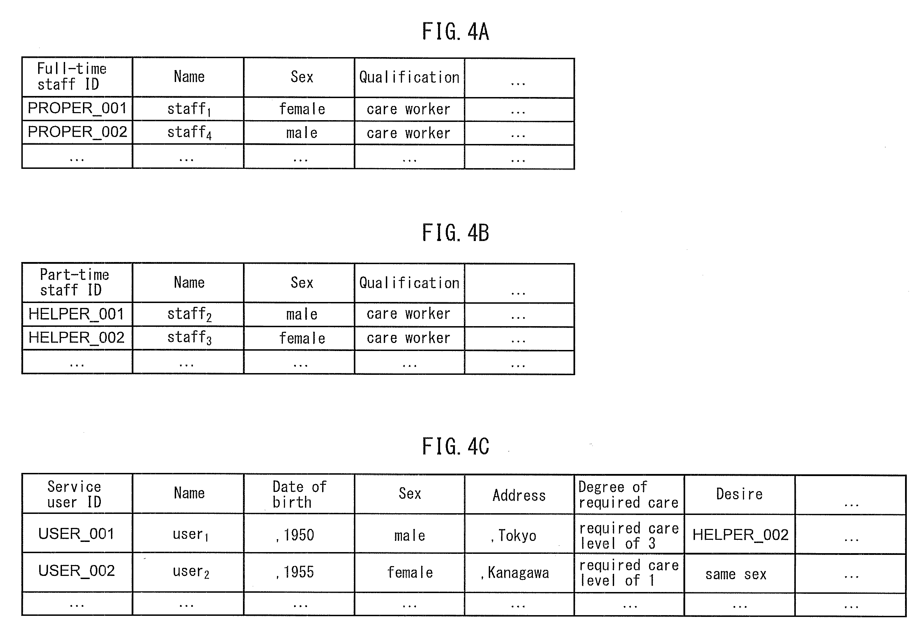

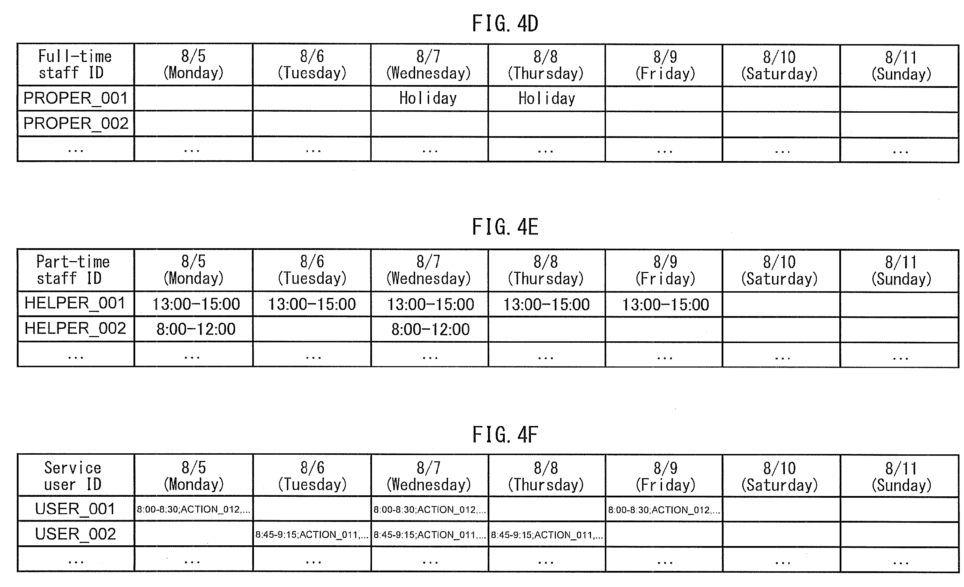

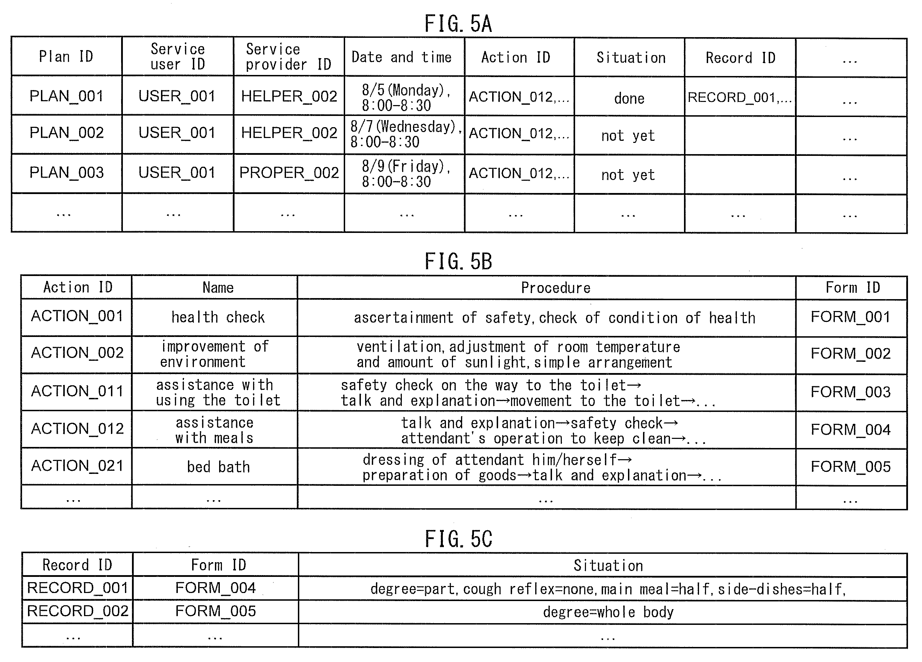

The server storage unit 32 includes at least one of, for example, a magnetic tape device, a magnetic disk device, and an optical disk device. The server storage unit 32 stores an operating system program, a driver program, an application program, data, etc., which are used in the processing by the server processing unit 33. For example, the server storage unit 32 performs creation of service scheduling or the like as the application program and stores a visiting service management support program or the like for creating display data relating to the results. Further, the server storage unit 32 stores, as data, a full-time staff attribute information management table (FIG. 4A) for managing full-time staff attribute information, a part-time staff attribute information management table (FIG. 4B) for managing part-time staff attribute information, a service user attribute information management table (FIG. 4C) for managing service user attribute information, a full-time staff's desired date and time management table (FIG. 4D) for managing the full-time staffs desired date and time of service provision, a part-time staffs desired date and time management table (FIG. 4E) for managing the part-time staffs desired date and time of service provision, a service user's desired date and time management table (FIG. 4F) for managing the service user's desired date and time of service use, a service scheduling management table (FIG. 5A) for managing restriction condition data when creating service scheduling and the service scheduling, a work procedure management table (FIG. 5B) for managing a service action work procedure, a service provision record management table (FIG. 5C) for managing service provision record input form data and a service provision record, etc. Furthermore, the server storage unit 32 may temporarily store temporary data relating to predetermined processing.

FIGs. 4A to 4F and FIGs. 5A to 5C are diagrams illustrating examples of data structures of various management tables.

In FIG. 4A, a full-time staff attribute information management table for managing full-time staff attribute information is illustrated. In the full-time staff attribute information management table, the identification number (ID), name, sex, qualification (e.g., care worker, care attendant, etc.), etc., of a full-time staff are stored for each full-time staff.

In the present embodiment, although "care worker" is displayed in the qualification field as an example of a visiting care service, for example, in the case of a visiting nursing service, "nurse" or the like is displayed in the qualification field.

In FIG. 4B, a part-time staff attribute information management table for managing part-time staff attribute information is illustrated. In the part-time staff attribute information management table, the ID, name, sex, qualification (e.g., care worker, care attendant, etc.), etc., of a part-time staff are stored for each part-time staff.

In FIG. 4C, a service user attribute information management table for managing service user attribute information is illustrated. In the service user attribute information management table, the ID, name, date of birth, sex, address, degree of required care (e.g., requiring assistance, required care level of 1 to 5, etc.), desire (e.g., desire regarding a staff or the like), etc., of a service user are stored for each service user.

In FIG. 4D, a full-time staffs desired date and time management table for managing the full-time staffs desired date and time of service provision is illustrated. In the full-time staffs desired date and time management table, the ID, date on which a full-time staff desires to take a holiday, etc., of a full-time staff are stored for each full-time staff.

In FIG. 4E, a part-time staffs desired date and time management table for managing the part-time staffs desired date and time of service provision is illustrated. In the part-time staffs desired date and time management table, the ID, desired date and time of service provision, etc., of a part-time staff are stored for each part-time staff.

In FIG. 4F, a service user's desired date and time management table for managing the service user's desired date and time of service use is illustrated. In the service user's desired date and time management table, the ID and desired date and time of service use, the ID of a service action to be used (e.g., assistance with using the toilet, assistance with meals, bed bath, etc.), etc., of a service user are stored for each service user.

In FIG. 5A, a service scheduling management table for managing service scheduling is illustrated. In the service scheduling management table, the ID of individual service scheduling, the ID of a service user relating to the individual service scheduling, the ID of a service provider, the service provision date and time, the ID of a service action to be provided, the service provision situation ("not yet" or "done"), the ID of a service provision record, etc., are stored for each individual service scheduling.

Although these pieces of information mainly relate to the schedule of the service user and the service provider, the present invention is not limited to those. Besides these pieces of information, for example, information relating to various plans, such as a flow of the entire business, may be included.

In FIG. 5B, a work procedure management table for managing the service action work procedure is illustrated. In the work procedure management table, the ID, name, and work procedure of a service action, the ID of a service provision record input form, etc., are stored for each service action.

In FIG. 5C, a service provision record management table for managing the service provision record is illustrated. In the service provision record management table, the ID of a service provision record, the ID of an input form, the service provision situation, etc., are stored for each service provision record.

The server processing unit 33 includes one or a plurality of processors and peripheral circuits thereof. The server processing unit 33 centralizedly controls the entire operation of the server 3 and is, for example, a CPU. The server processing unit 33 controls the operation of the server communication unit 31 or the like so that the various kinds of processing of the server 3 are performed by an appropriate procedure in accordance with programs stored in the server storage unit 32, requests from the mobile terminal 2, etc. The server processing unit 33 performs processing based on the programs (operating system program, driver program, application program, etc.) stored in the server storage unit 32. Further, the server processing unit 33 may execute a plurality of programs (application program or the like) in parallel.

### (2.2.1) Function of server processing unit 33

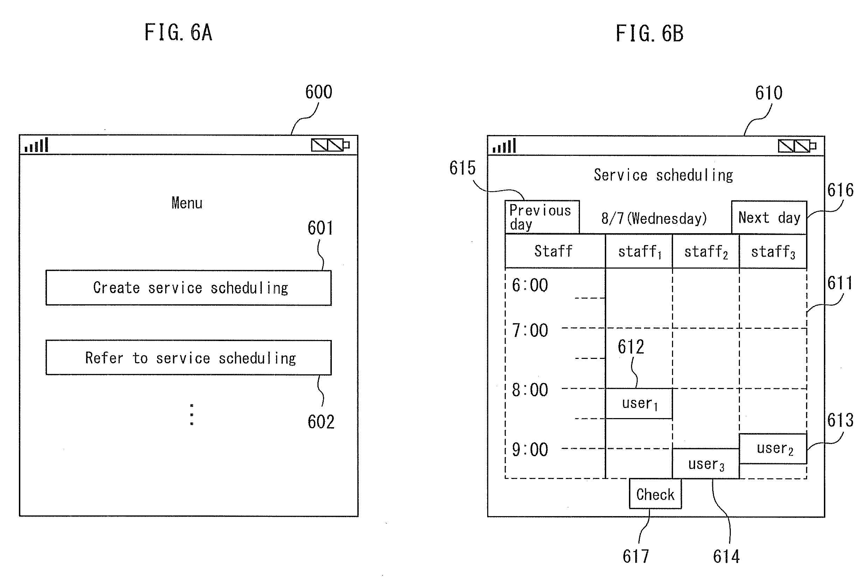

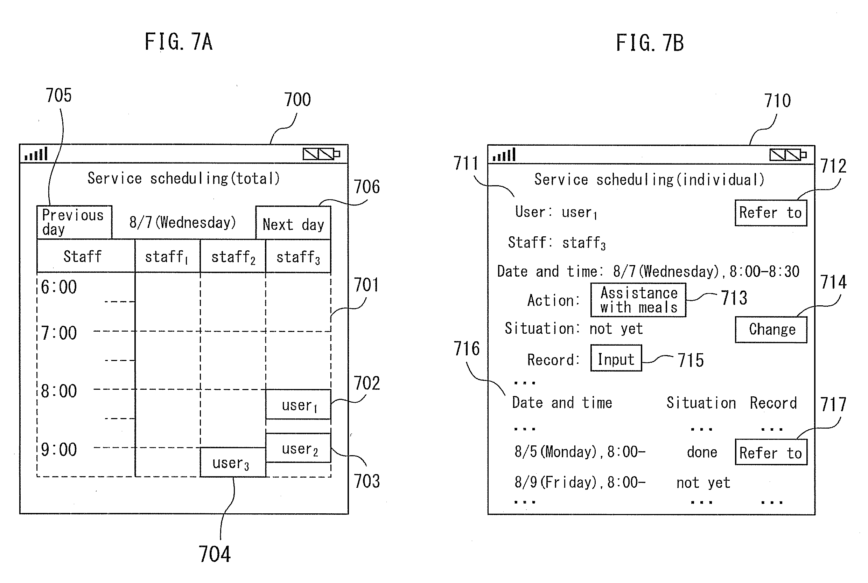

FIGs. 6A to 6D and FIGs. 7A to 7D are diagrams illustrating examples of the display screen of the mobile terminal 2 based on the display data created by the server 3.

In FIG. 6A, a home screen 600 that is displayed when a visiting service management support service is started is illustrated. The home screen 600 is displayed based on home screen display data received from the server 3.

At the center of the screen, buttons 601 and 602 are displayed. The server 3 is requested via the terminal communication unit 21 to create service scheduling when the "Create service scheduling" button 601 is pressed down, and to refer to service scheduling when the "Refer to service scheduling" button 602 is pressed down.

In FIG. 6B, a Service scheduling editing screen 610 that is displayed when a request to create service scheduling is made on the home screen 600 illustrated in FIG. 6A is illustrated. The Service scheduling editing screen 610 is displayed based on service scheduling editing screen display data received from the server 3.

At the center of the screen, service scheduling 611 for one day is displayed in the form of a table in which the horizontal axis represents the service provider and the vertical axis represents the service provision time, and within the table, icons 612 to 614 each indicating the individual service scheduling for a service user are displayed. When one of the icons 612 to 614 is pressed down, the server 3 is requested via the terminal communication unit 21 to refer to service user attribute information using the ID of the service user relating to the corresponding individual service scheduling as a parameter. The service user relating to the corresponding individual service scheduling may be changed, by moving among the icons 612 to 614.

Further, buttons 615 and 616 are displayed at the top of the screen. The server 3 is requested via the terminal communication unit 21 to refer to the service scheduling 611 for the previous day when the "Previous day" button 615 is pressed down, and to refer to the service scheduling 611 for the next day when the "Next day" button 616 is pressed down, respectively, using the corresponding date as a parameter.

Furthermore, a "Check" button 617 is displayed at the bottom of the screen, and when this button is pressed down, the server 3 is requested via the terminal communication unit 21 to store the service scheduling in the server storage unit 32.

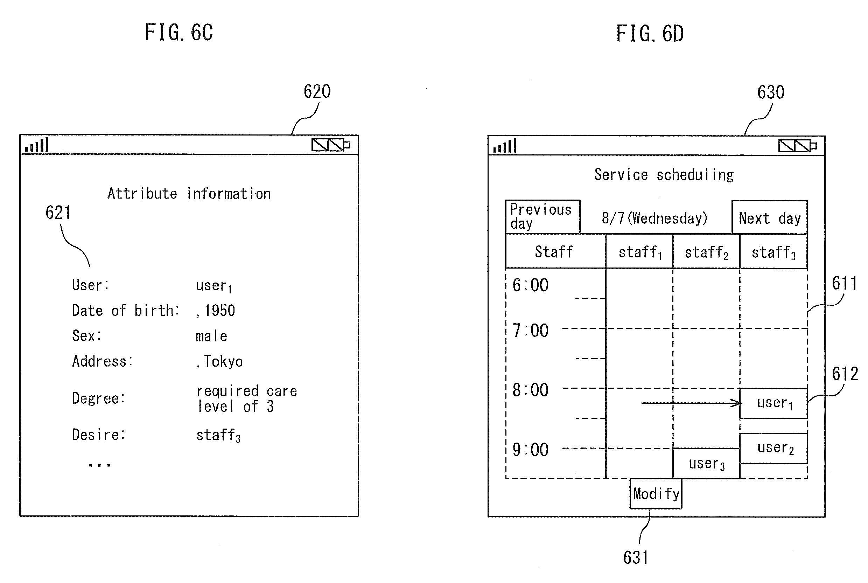

In FIG. 6C, an Attribute information display screen 620 that is displayed when a request to refer to the service user attribute information is made on the Service scheduling editing screen 610 illustrated in FIG. 6B is illustrated. The Attribute information display screen 620 is displayed based on attribute information display screen display data received from the server 3.

At the center of the screen, service user attribute information 621 is displayed for each item.

In FIG. 6D, a Service scheduling editing screen 630 that is displayed when the service scheduling 611 is edited on the Service scheduling editing screen 610 illustrated in FIG. 6B is illustrated. The service provider relating to the individual service scheduling for service user 1 is changed from staff 1 to staff 3. Further, triggered by this change, the "Check" button 617 is changed to a "Modify" button 631.

When the "Modify" button 631 is pressed down, the server 3 is requested via the terminal communication unit 21 to modify the service scheduling using the ID of the individual service scheduling relating to the change and the ID of the service provider as parameters.

Although the screens for the manager who creates the service scheduling are illustrated in FIGs. 6A to 6D, for example, if the login is performed using the ID of a service provider, only the service scheduling of which the service provider him/herself is in charge may be displayed on the screen, without displaying the service scheduling of the other service providers. This also applies in the following explanation.

In FIG. 7A, a Service scheduling (total) display screen 700 that is displayed when a request to refer to the service scheduling is made on the home screen 600 illustrated in FIG. 6A is illustrated. The Service scheduling (total) display screen 700 is displayed based on service scheduling display screen display data received from the server 3.

At the center of the screen, service scheduling 701 for one day is displayed in the form of a table in which the horizontal axis represents the service provider and the vertical axis represents the service provision time, and within the table, icons 702 to 704 each indicating the individual service scheduling for a service user are displayed. When one of the icons 702 to 704 is pressed down, the server 3 is requested via the terminal communication unit 21 to refer to the individual service scheduling using the ID of the corresponding individual service scheduling as a parameter.

Further, buttons 705 and 706 are displayed at the top of the screen. The server 3 is requested via the terminal communication unit 21 to refer to the service scheduling 701 for the previous day when the "Previous day" button 705 is pressed down, and to refer to the service scheduling 701 for the next day when the "Next day" button 706 is pressed down, respectively, using the corresponding date as a parameter.

In FIG. 7B, a Service scheduling (individual) display screen 710 that is displayed when a request to refer to the individual service scheduling is made on the Service scheduling (total) display screen 700 illustrated in FIG. 7A is illustrated. The Service scheduling (individual) display screen 710 is displayed based on individual service scheduling display screen display data received from the server 3.

At the center of the screen, individual service scheduling 711 is displayed for each item and for some of the items, buttons 712 to 715 are displayed. When the "Refer to" button 712 is pressed down, the server 3 is requested via the terminal communication unit 21 to refer to the service user attribute information using the ID of the corresponding service user as a parameter. When the "Assistance with meals" button 713 is pressed down, the server 3 is requested via the terminal communication unit 21 to refer to the service action work procedure using the ID of the corresponding service action as a parameter. When the "Change" button 714 is pressed down, the server 3 is requested via the terminal communication unit 21 to change the service provision situation from "not yet" to "done" using the ID of the individual service scheduling 711 as a parameter. Further, when the "Input" button 715 is pressed down, the server 3 is requested via the terminal communication unit 21 to transmit the service provision record input form using the ID of the individual service scheduling 711 as a parameter.

At the bottom of the screen, another individual service scheduling 716 for the service user relating to the individual service scheduling 711 is listed for each item and for some of the items, a "Refer to" button 717 is displayed. When the "Refer to" button 717 is pressed down, the server 3 is requested via the terminal communication unit 21 to refer to the service provision record using the ID of the corresponding individual service scheduling as a parameter.

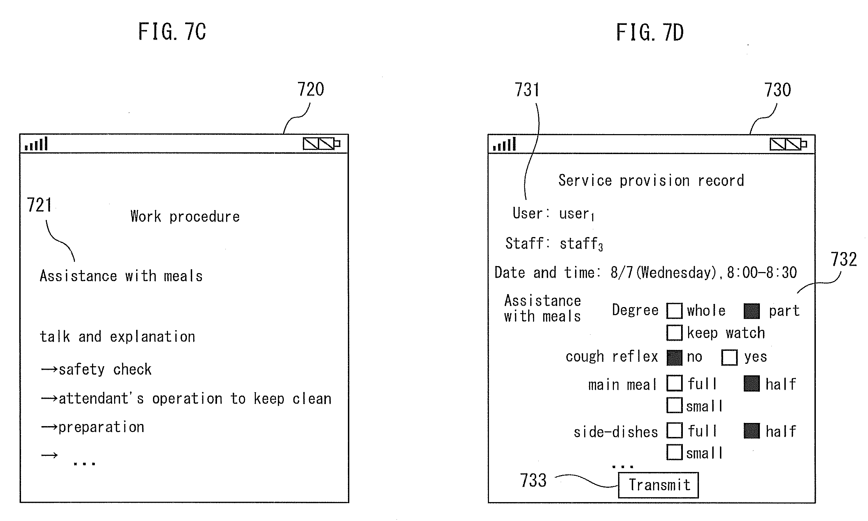

In FIG. 7C, a Work procedure display screen 720 that is displayed when a request to refer to the service action work procedure is made on the Service scheduling (individual) display screen 710 illustrated in FIG. 7B is illustrated. The Work procedure display screen 720 is displayed based on work procedure display screen display data received from the server 3.

At the center of the screen, a service action work procedure 721 is displayed in order.

In FIG. 7D, a Service provision record input screen 730 that is displayed when a request to transmit the service provision record input form is made on the Service scheduling (individual) display screen 710 illustrated in FIG. 7B is illustrated. The Service provision record input screen 730 is displayed based on service provision record input screen display data received from the server 3.

At the top of the screen, individual service scheduling 731 is displayed for each item.

Further, a plurality of input boxes 732, such as a checkbox, a radio button, and a text area, is displayed at the center of the screen, for each service action and, a server provision record may be input, by checking the relevant input box.

Furthermore, a "Transmit" button 733 is displayed at the bottom of the screen, and when this button is pressed down, the server 3 is requested via the terminal communication unit 21 to store the service provision record in the server storage unit 32 using the ID of the individual service scheduling 731, the data input to the input box 732, and the ID of the corresponding input form as parameters.

### (2.2.2) Configuration of server processing unit 33

In order to implement the above functions, the server processing unit 33 includes a service scheduling information creation unit 331, a service scheduling information reference unit 332, a service scheduling information updating unit 333, a service user management information reference unit 334, and a service user management information updating unit 335. Each of these units is a function module that is implemented by a program executed by the processor included in the server processing unit 33. Alternatively, each of these units may be mounted on the server 3 as firmware.

Hereinafter, the processing by the service scheduling information creation unit 331 is explained. The service scheduling information creation unit 331 creates and presents service scheduling to the manager. If the service scheduling is modified by the manager, the service scheduling information creation unit 331 creates service scheduling again so that the modification is reflected and presents the service scheduling to the manager. Then, after the service scheduling is checked by the manager, the service scheduling information creation unit 331 stores the service scheduling in the server storage unit 32.

Specifically, if the service scheduling information creation unit 331 receives a request to create service scheduling from the mobile terminal 2 via the server communication unit 31, extracts the service user's desired date and time of service use. For example, if service scheduling for the next one week is created, the service scheduling information creation unit 331 extracts information on the desired date and time of service use for one week. The period of time for which service scheduling is created may be based on a value determined in advance, or the period may be specified each time service scheduling is created.

In other words, the service scheduling information creation unit 331 refers to the service user's desired date and time management table stored in the server storage unit 32 and extracts the service user's desired date and time of service use for a predetermined period of time.

The service scheduling information creation unit 331 extracts the part-time staff's desired date and time of service provision.

In other words, the service scheduling information creation unit 331 refers to the part-time staff's desired date and time management table stored in the server storage unit 32 and extracts the part-time staff's desired date and time of service provision the predetermined period of time of which is the same as that of the service user's each desired date and time of service use for each part-time staff.

The service scheduling information creation unit 331 creates part-time staff service scheduling based on the extracted service user's desired date and time of service user and the extracted part-time staffs desired date and time of service provision.

In other words, the service scheduling information creation unit 331 compares the extracted service user's each desired date and time of service use with the similarly extracted part-time staff's each desired date and time of service provision, and then, assigns the time period where both the desired date and times coincide with each other, and temporarily stores the results (combination of the ID of the service user, the ID of the part-time staff, and the date and time of service provision) in the server storage unit 32 after giving an individual service scheduling ID newly adopted.

The assignment of the service provider to the service user is generally known as a nurse scheduling problem and a variety of solutions have been proposed. For example, if the service provider is replaced with a production machine and the service user with a lot, which is the minimum unit of production, the nurse scheduling may be regarded as parallel machine scheduling, and the solutions using the optimization method, such as the branch and bound method, the neutral network, and the genetic algorithm, have been proposed (e.g., "Genetic Algorithm Approach to an Operation Assignment Problem", Taima Junji and two other authors, Transactions of the Institute of Systems, Control and Information Engineers, the Institute of Systems, Control and Information Engineers, October 15, 1994, Vol. 7, No. 10, pp. 433-435). Consequently, explanation of details of the processing to assign the service provider to the service user is omitted.

The service scheduling information creation unit 331 creates the service scheduling editing screen display data based on the created part-time staff service scheduling.

In other words, the service scheduling information creation unit 331 refers to the part-time staff service scheduling stored in the server storage unit 32 using a predetermined date as a key and specifies the corresponding individual service scheduling. Further, the service scheduling information creation unit 331 refers to the service user attribute information management table stored in the server storage unit 32 using the ID of the service user relating to the specified individual service scheduling as a key and extracts the name of the corresponding service user. Furthermore, the service scheduling information creation unit 331 refers to the part-time staff attribute information management table stored in the server storage unit 32 using the ID of the part-time staff relating to the specified individual service scheduling as a key and extracts the name of the corresponding part-time staff. Then, the service scheduling information creation unit 331 creates the service scheduling editing screen display data for the specified individual service scheduling, including the ID of the individual service scheduling, the ID of the service user relating to the individual service scheduling, the ID of the part-time staff, etc., and for displaying the extracted name of the service user, the extracted name of the part-time staff, the service provision time relating to the individual service scheduling, icons and buttons for receiving various instructions, etc., in a predetermined layout.

The service scheduling information creation unit 331 transmits the created service scheduling editing screen display data to the mobile terminal 2 via the server communication unit 31.

If the service scheduling information creation unit 331 receives a request to refer to the service scheduling of the specified date from the mobile terminal 2 via the server communication unit 31, the service scheduling information creation unit 331 creates the service scheduling editing screen display data based on the created part-time staff service scheduling.

In other words, the service scheduling information creation unit 331 refers to the part-time staff service scheduling stored in the server storage unit 32 using the date given as a parameter as a key and specifies the corresponding individual service scheduling. Then, the service scheduling information creation unit 331 creates the service scheduling editing screen display data based on the specified individual service scheduling by the same procedure as that described above.

On the other hand, if the service scheduling information creation unit 331 receives a request to modify the service scheduling, the service scheduling information creation unit 331 creates the part-time staff service scheduling again based on the extracted service user's desired date and time of service use and the extracted part-time staffs desired date and time of service provision so that the modification is reflected.

In other words, the service scheduling information creation unit 331 sets a combination of the service user and the date and time of service provision (desired date and time of service use) relating to the individual service scheduling corresponding to the ID given as a parameter and the service provider corresponding to the ID given similarly as a parameter as a restriction condition, and assigns the extracted part-time staff's each desired date and time of service provision to the similarly extracted service user's each desired date and time of service use so that the restriction condition is met.

On the other hand, if the service scheduling information creation unit 331 receives a request to store the service scheduling, the service scheduling information creation unit 331 stores the created part-time staff service scheduling in the server storage unit 32.

In other words, the service scheduling information creation unit 331 refers to the part-time staff service scheduling stored in the server storage unit 32 and specifies the individual service scheduling. Further, the service scheduling information creation unit 331 refers to the service user's desired date and time management table stored in the server storage unit 32 using the ID of the service user and the date and time of service provision relating to the specified individual service scheduling and extracts the ID of the corresponding service action to be used. Then, the service scheduling information creation unit 331 stores the ID of the specified individual service scheduling, the ID of the service user relating to the individual service scheduling, the ID of the service provider, the date and time of service provision, the extracted ID of the service action to be used, the service provision situation ("not yet"), the ID of the service provision record ("none"), etc., in the service scheduling management table stored in the server storage unit 32.

Subsequently, the service scheduling information creation unit 331 specifies the date and time of service provision that is not the date and time of service provision relating to the created part-time staff service scheduling (hereinafter, referred to as "service user's unassigned desired date and time of service use") among the extracted service user's desired date and times of service use.

The service scheduling information creation unit 331 extracts the full-time staff's desired date and time of service provision.

In other words, the service scheduling information creation unit 331 refers to the full-time staffs desired date and time management table stored in the server storage unit 32 and extracts the full-time staff's desired date and time of service provision the predetermined period of time of which is the same as that of the service user's each desired date and time of service use, or during the period of time in which the unassigned desired date and time of service use is included.

The service scheduling information creation unit 331 creates the full-time staff service scheduling based on the specified service user's unassigned desired date and time of service use and the extracted full-time staffs desired date and time of service provision.

In other words, the service scheduling information creation unit 331 compares the specified service user's each unassigned desired date and times of service use with the extracted full-time staffs each desired date and time of service provision, and assigns the time period where both the desired date and times coincide with each other, and temporarily stores the results in the server storage unit 32 after giving an individual service scheduling ID newly adopted.

The service scheduling information creation unit 331 creates the service scheduling editing screen display data based on the created full-time staff service scheduling.

In other words, the service scheduling information creation unit 331 refers to the full-time staff service scheduling stored in the server storage unit 32 using a predetermined date as a key and specifies the corresponding individual service scheduling. Then, the service scheduling information creation unit 331 creates the service scheduling editing screen display data based on the specified individual service scheduling by the same procedure as that described above.

The service scheduling information creation unit 331 transmits the created service scheduling editing screen display data to the mobile terminal 2 via the server communication unit 31.

If the service scheduling information creation unit 331 receives a request to refer to the service scheduling of the specified date from the mobile terminal 2 via the server communication unit 31, the service scheduling information creation unit 331 creates the service scheduling editing screen display data based on the created full-time staff service scheduling.

In other words, the service scheduling information creation unit 331 refers to the full-time staff service scheduling stored in the server storage unit 32 using the date given as a parameter as a key and specifies the corresponding individual service scheduling. Then, the service scheduling information creation unit 331 creates the service scheduling editing screen display data based on the specified individual service scheduling by the same procedure as that described above.

On the other hand, if the service scheduling information creation unit 331 receives a request to modify the service scheduling, the service scheduling information creation unit 331 creates the full-time staff service scheduling again based on the specified service user's unassigned desired date and time of service use and the extracted full-time staff's desired date and time of service provision so that the modification is reflected.

In other words, the service scheduling information creation unit 331 sets the contents of the modification as a restriction condition and assigns the extracted full-time staff's each desired date and time of service provision to the specified service user's each unassigned desired date and time of service use so that the restriction condition is met.

On the other hand, if the service scheduling information creation unit 331 receives a request to store the service scheduling, the service scheduling information creation unit 331 stores the created full-time staff service scheduling in the server storage unit 32.

In other words, the service scheduling information creation unit 331 refers to the full-time staff service scheduling stored in the server storage unit 32 and specifies the individual service scheduling. Then, the service scheduling information creation unit 331 stores the specified individual service scheduling or the like in the service scheduling management table stored in the server storage unit 32 by the same procedure as that described above.

For example, if the full-time staff's desired date and time management table illustrated in FIG. 4D, the part-time staff's desired date and time management table illustrated in FIG. 4E, and the service user's desired date and time management table illustrated in FIG. 4F are given by the above processing, the service scheduling management table illustrated in FIG. 5A is created as a result.

Hereinafter, the processing by the service scheduling information reference unit 332 is explained. The service scheduling information reference unit 332 refers to and presents the service scheduling to the service provider.

Specifically, if the service scheduling information reference unit 332 receives a request to refer to the service scheduling from the mobile terminal 2 via the server communication unit 31, the service scheduling information reference unit 332 creates the service scheduling display screen display data based on the service scheduling.

In other words, the service scheduling information reference unit 332 refers to the service scheduling management table stored in the server storage unit 32 using a predetermined date as a key and specifies the corresponding individual service scheduling. Further, the service scheduling information reference unit 332 refers to the service user attribute information management table stored in the server storage unit 32 using the ID of the service user relating to the specified individual service scheduling and extracts the name of the corresponding service user. Furthermore, the service scheduling information reference unit 332 refers to the full-time staff attribute information management table and the part-time staff attribute information management table that are stored in the server storage unit 32 using the ID of the service provider relating to the specified individual service scheduling as a key and extracts the name of the corresponding service provider. Then, the service scheduling information reference unit 332 creates the service scheduling display screen display data for the specified individual service scheduling, including the ID of the individual service scheduling or the like, and for displaying the extracted name of the service user, the extracted name of the service provider, the service provision time relating to the individual service scheduling, icons and buttons for receiving various instructions, etc., in a predetermined layout. FIG. 7A is an example of a service scheduling screen display.

The service scheduling information reference unit 332 transmits the created service scheduling display screen display data to the mobile terminal 2 via the server communication unit 31.

If the service scheduling information reference unit 332 receives a request to refer to the service scheduling of the specified date from the mobile terminal 2 via the server communication unit 31, the service scheduling information reference unit 332 creates the service scheduling display screen display data based on the service scheduling.

In other words, the service scheduling information reference unit 332 refers to the service scheduling management table stored in the server storage unit 32 using the date given as a parameter as a key and specifies the corresponding individual service scheduling. Then, the service scheduling information reference unit 332 creates the service scheduling display screen display data based on the specified individual service scheduling by the same procedure as that described above.

On the other hand, if the service scheduling information reference unit 332 receives a request to refer to the individual service scheduling, the service scheduling information reference unit 332 creates the individual service scheduling display screen display data based on the individual service scheduling.

In other words, the service scheduling information reference unit 332 refers to the service scheduling management table stored in the server storage unit 32 using the ID of the individual service scheduling given as a parameter as a key and specifies the corresponding individual service scheduling. Further, the service scheduling information reference unit 332 refers to the service user attribute information management table stored in the server storage unit 32 using the ID of the service user relating to the specified individual service scheduling as a key and extracts the name of the corresponding service user. Furthermore, the service scheduling information reference unit 332 refers to the full-time staff attribute information management table and the part-time staff attribute information management table that are stored in the server storage unit 32 using the ID of the service provider relating to the specified individual service scheduling as a key and extracts the name of the corresponding service provider. Furthermore, the service scheduling information reference unit 332 refers to the work procedure management table stored in the server storage unit 32 using the ID of the service action to be provided relating to the specified individual service scheduling as a key and extracts the name of the corresponding service action to be provided. Then, the service scheduling information reference unit 332 creates the individual service scheduling display screen display data for the specified individual service scheduling, including the ID of the individual service scheduling, the ID of the service user relating to the individual service scheduling, the ID of the service action to be provided, etc., and for displaying the extracted name of the service user, the extracted name of the service provider, the extracted name of the service action to be provided, the service provision date and time relating to the individual service scheduling, the service provision situation, icons and buttons for receiving various instructions, etc., in a predetermined layout. FIG. 7B is an example of an individual service scheduling screen display.

Hereinafter, the processing by the service scheduling information updating unit 333 is explained. The service scheduling information updating unit 333 provides a unit configured to update service scheduling to the service provider.

Specifically, if the service scheduling information updating unit 333 receives a request to change the service provision situation from the mobile terminal 2 via the server communication unit 31, the service scheduling information updating unit 333 changes the service provision situation relating to the individual service scheduling.

In other words, the service scheduling information updating unit 333 refers to the service scheduling management table stored in the server storage unit 32 using the ID of the individual service scheduling given as a parameter as a key and specifies the corresponding individual service scheduling. Then, the service scheduling information updating unit 333 changes the service provision situation relating to the specified individual service scheduling from "not yet" to "done". For example, as illustrated in FIG. 7B, when the "Change" button 714 is pressed down, the service provision situation is changed from "not yet" to "done", instructions to change the service provision situation are transmitted to the server 3, and the data of the service provision situation is updated.

Hereinafter, the processing by the service user management information reference unit 334 is explained. The service user management information reference unit 334 refers to and presents the service user attribute information or the like to the service provider.

Specifically, if the service user management information reference unit 334 receives a request to refer to the service user attribute information from the mobile terminal 2 via the server communication unit 31, the service user management information reference unit 334 creates the attribute information display screen display data.

In other words, the service user management information reference unit 334 refers to the service user attribute information management table stored in the server storage unit 32 using the ID of the service user given as a parameter as a key and extracts the corresponding service user attribute information. Then, the service user management information reference unit 334 creates the attribute information display screen display data for displaying the extracted service user attribute information or the like in a predetermined layout. FIG. 6C is an example of a service user attribute information screen display.

On the other hand, if the service user management information reference unit 334 receives a request to refer to the service action work procedure, the service user management information reference unit 334 creates the work procedure display screen display data.

In other words, the service user management information reference unit 334 refers to the work procedure management table stored in the server storage unit 32 using the ID of the service action given as a parameter as a key and extracts the name and the work procedure of the corresponding service action. Then, the service user management information reference unit 334 creates the work procedure display screen display data for displaying the extracted name and work procedure of the service action or the like in a predetermined layout. FIG. 7C is an example of a work procedure information screen display.

On the other hand, if the service user management information reference unit 334 receives a request to refer to the service provision record, the service user management information reference unit 334 creates service provision record display screen display data.

In other words, the service user management information reference unit 334 refers to the service scheduling management table stored in the server storage unit 32 using the ID of the individual service scheduling given as a parameter as a key and specifies the corresponding individual service scheduling. Further, the service user management information reference unit 334 refers to the service user attribute information management table stored in the server storage unit 32 using the ID of the service user relating to the specified individual service scheduling as a key and extracts the name of the corresponding service user. Furthermore, the service user management information reference unit 334 refers to the full-time staff attribute information management table and the part-time staff attribute information management table that are stored in the server storage unit 32 using the ID of the service provider relating to the specified individual service scheduling and extracts the name of the corresponding service provider. Furthermore, the service user management information reference unit 334 extracts the ID of the service provision record relating to the specified individual service scheduling. Furthermore, the service user management information reference unit 334 refers to the service provision record management table stored in the server storage unit 32 using the ID of the extracted service provision record as a key and extracts the ID of the corresponding input form and the service provision situation. Furthermore, the service user management information reference unit 334 acquires the input form data corresponding to the extracted ID of the input form from the server storage unit 32. Then, the service user management information reference unit 334 creates the service provision record display screen display data for displaying the extracted name of the service user, the extracted name of the service provider, the service provision date and time relating to the specified individual service scheduling, the extracted service provision situation, etc., in a predetermined layout by using the acquired input form data.

The service user management information reference unit 334 transmits the created attribute information display screen display data or the like to the mobile terminal 2 via the server communication unit 31.

Hereinafter, the processing by the service user management information updating unit 335 is explained. The service user management information updating unit 335 provides a unit configured to update the service user management information to the service provider.

Specifically, if the service user management information updating unit 335 receives a request to transmit the service provision record input form from the mobile terminal 2 via the server communication unit 31, the service user management information updating unit 335 creates the service provision record input screen display data.

In other words, the service user management information updating unit 335 refers to the service scheduling management table stored in the server storage unit 32 using the ID of the individual service scheduling given as a parameter as a key and specifies the corresponding individual service scheduling. Further, the service user management information updating unit 335 refers to the service user attribute information management table stored in the server storage unit 32 using the ID of the service user relating to the specified individual service scheduling as a key and extracts the name of the corresponding service user. Furthermore, the service user management information updating unit 335 refers to the full-time staff attribute information management table and the part-time staff attribute information management table that are stored in the server storage unit 32 using the ID of the service provider relating to the specified individual service scheduling and extracts the name of the corresponding service provider. Furthermore, the service user management information updating unit 335 extracts the ID of the service action to be provided relating to the specified individual service scheduling. Furthermore, the service user management information updating unit 335 refers to the work procedure management table stored in the server storage unit 32 using the ID of the extracted service action to be provided as a key and extracts the ID of the corresponding service provision record input form. Furthermore, the service user management information updating unit 335 acquires the input form data corresponding to the extracted ID of the input form from the server storage unit 32. Then, the service user management information updating unit 335 creates the service provision record input screen display data including the ID of the given individual service scheduling, the extracted ID of the input form, etc., and for displaying the extracted name of the service user, the extracted name of the service provider, the service provision date and time relating to the specified individual service scheduling, the input boxes, etc., in a predetermined layout by using the acquired input form data. FIG. 7D is an example of a service provision record input form screen display.

The service user management information updating unit 335 transmits the created service provision record input screen display data to the mobile terminal 2 via the server communication unit 31.

If the service user management information updating unit 335 receives a request to store the service provision record from the mobile terminal 2 via the server communication unit 31, the service user management information updating unit 335 stores the service provision record in the server storage unit 32.

In other words, the service user management information updating unit 335 refers to the service scheduling management table stored in the server storage unit 32 using the ID of the individual service scheduling given as a parameter as a key and specifies the corresponding individual service scheduling. Further, the service user management information updating unit 335 stores the ID of the input form similarly given as a parameter and the data input to the input boxes (service provision situation) in the service provision record management table stored in the server storage unit 32 after giving a service provision record ID newly adopted. Then, the service user management information updating unit 335 stores the given service provision record ID as the ID of the service provision record relating to the specified individual service scheduling.

### (3) Operation of server 3

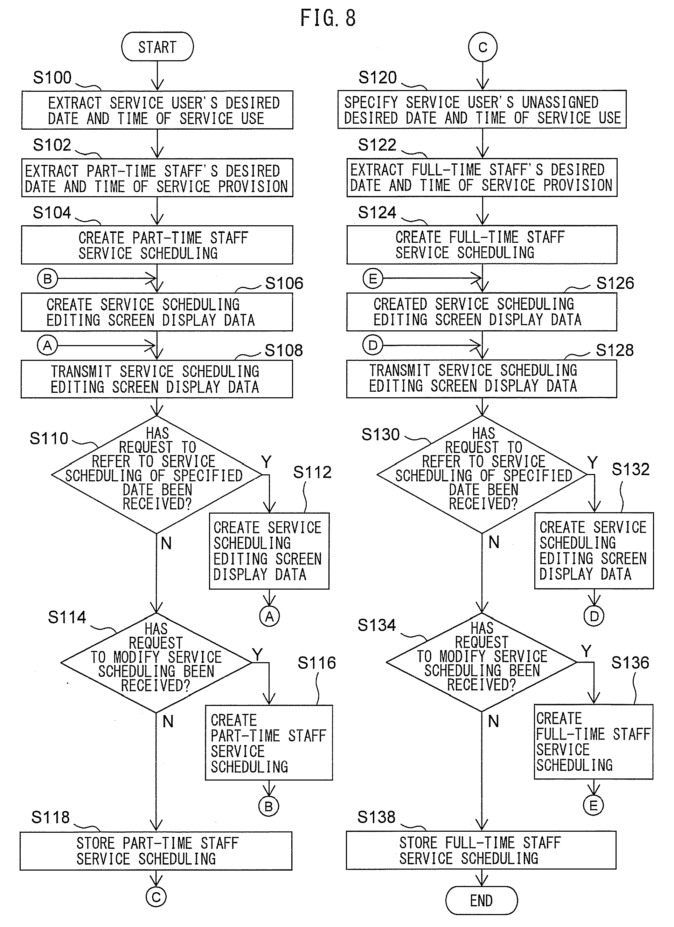

FIG. 8, FIGs. 9A and 9B, and FIGs. 10A and 10B are diagrams illustrating an example of an operation flow of the server 3. The operation flow to be explained in the following is performed mainly by the server processing unit 33 in cooperation with each element of the server 3 based on the programs stored in the server storage unit 32 in advance.

FIG. 8 is a diagram illustrating an example of an operation flow of the service scheduling information creation unit 331.

If the service scheduling information creation unit 331 receives a request to create service scheduling from the mobile terminal 2 via the server communication unit 31, the service scheduling information creation unit 331 extracts the service user's desired date and time of service use (step S100).

The service scheduling information creation unit 331 extracts the part-time staffs desired date and time of service provision (step S102).

The service scheduling information creation unit 331 creates the part-time staff service scheduling based on the extracted service user's desired date and time of service use and the extracted part-time staffs desired date and time of service provision (step S104).

The service scheduling information creation unit 331 creates the service scheduling editing screen display data based on the created part-time staff service scheduling (step S106).

The service scheduling information creation unit 331 transmits the created service scheduling editing screen display data to the mobile terminal 2 via the server communication unit 31 (step S108).

If the service scheduling information creation unit 331 receives a request to refer to the service scheduling of the specified date from the mobile terminal 2 via the server communication unit 31 (step S110 - Yes), the service scheduling information creation unit 331 creates the service scheduling editing screen display data based on the created part-time staff service scheduling (step S112). Then, the service scheduling information creation unit 331 returns to the transmission processing of the service scheduling editing screen display data (step S108).

On the other hand, if the service scheduling information creation unit 331 receives a request to modify the service scheduling (step S114 - Yes), the service scheduling information creation unit 331 creates the part-time staff service scheduling again based on the extracted service user's desired date and time of service use and the extracted part-time staff's desired date and time of service provision so that the modification is reflected ((step S116). Then, the service scheduling information creation unit 331 returns to the creation processing of the service scheduling editing screen display data (step S106).

On the other hand, if the service scheduling information creation unit 331 receives a request to store the service scheduling (step S114 - No), the service scheduling information creation unit 331 stores the created part-time staff service scheduling in the server storage unit 32 (step S118).

The service scheduling information creation unit 331 specifies the service user's unassigned desired date and time of service use (step S120).

The service scheduling information creation unit 331 extracts the full-time staffs desired date and time of service provision (step S122).

The service scheduling information creation unit 331 creates the full-time staff service scheduling based on the specified service user's unassigned desired date and time of service use and the extracted full-time staffs desired date and time of service provision (step S124).

The service scheduling information creation unit 331 creates the service scheduling editing screen display data based on the created full-time staff service scheduling (step S126).

The service scheduling information creation unit 331 transmits the created service scheduling editing screen display data to the mobile terminal 2 via the server communication unit 31 (step S128).

If the service scheduling information creation unit 331 receives a request to refer to the service scheduling of the specified date from the mobile terminal 2 via the server communication unit 31 (step S130 - Yes), the service scheduling information creation unit 331 creates the service scheduling editing screen display data based on the created full-time staff service scheduling (step S132). Then, the service scheduling information creation unit 331 returns to the transmission processing of the service scheduling editing screen display data (step S128).

On the other hand, if the service scheduling information creation unit 331 receives a request to modify the service scheduling (step S134 - Yes), the service scheduling information creation unit 331 creates the full-time staff service scheduling again based on the specified service user's unassigned desired date and time of service use and the extracted full-time staff's desired date and time of service provision so that the modification is reflected (step S136). Then, the service scheduling information creation unit 331 returns to the creation processing of the service scheduling editing screen display data (step S126).

On the other hand, if the service scheduling information creation unit 331 receives a request to store the service scheduling (step S134 - No), the service scheduling information creation unit 331 stores the created full-time staff service scheduling in the server storage unit 32 (step S138). Then, the service scheduling information creation unit 331 terminates the processing.

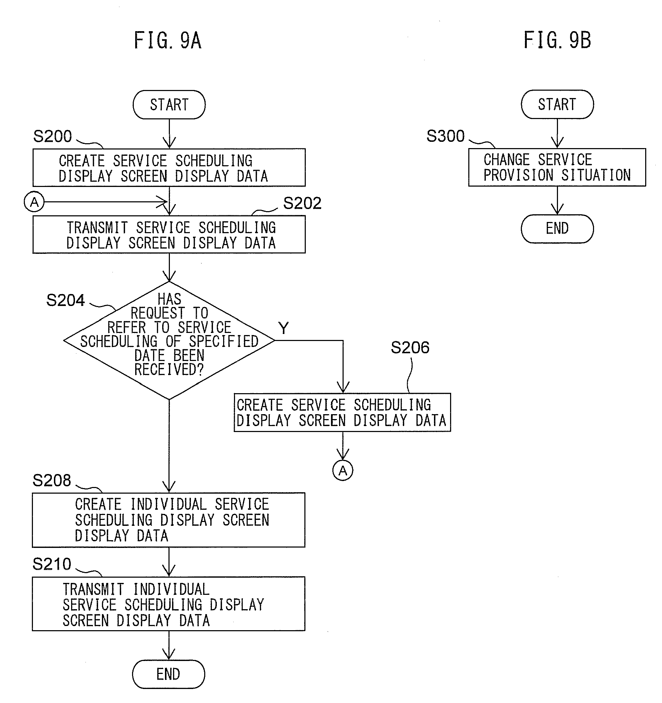

FIG. 9A is a diagram illustrating an example of an operation flow of the service scheduling information reference unit 332.

If the service scheduling information reference unit 332 receives a request to refer to the service scheduling from the mobile terminal 2 via the server communication unit 31, the service scheduling information reference unit 332 creates the service scheduling display screen display data based on the service scheduling (step S200).

The service scheduling information reference unit 332 transmits the created service scheduling display screen display data to the mobile terminal 2 via the server communication unit 31 (step S202).

If the service scheduling information reference unit 332 receives a request to refer to the service scheduling of the specified date from the mobile terminal 2 via the server communication unit 31 (step S204 - Yes), the service scheduling information reference unit 332 creates the service scheduling display screen display data based on the service scheduling (step S206). Then, the service scheduling information reference unit 332 returns to the transmission processing of the service scheduling display screen display data (step S202).

On the other hand, if the service scheduling information reference unit 332 receives a request to refer to the individual service scheduling (step S204 - No), the service scheduling information reference unit 332 creates the individual service scheduling display screen display data based on the individual service scheduling (step S208).

The service scheduling information reference unit 332 transmits the created individual service scheduling display screen display data to the mobile terminal 2 via the server communication unit 31 (step S210). Then, the service scheduling information reference unit 332 terminates the processing.

FIG. 9B is a diagram illustrating an example of an operation flow of the service scheduling information updating unit 333.

If the service scheduling information updating unit 333 receives a request to change the service provision situation from the mobile terminal 2 via the server communication unit 31, the service scheduling information updating unit 333 changes the service provision situation relating to the individual service scheduling (step S300). Then, the service scheduling information updating unit 333 terminates the processing.

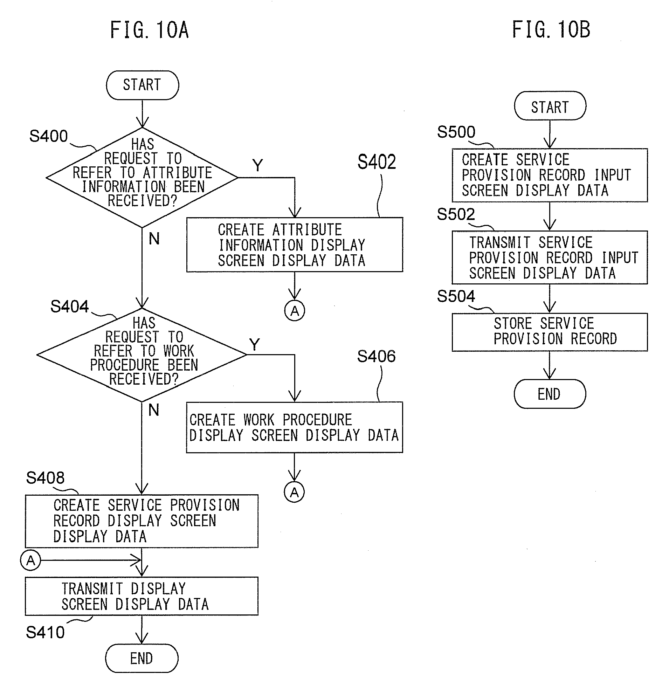

FIG. 10A is a diagram illustrating an example of an operation flow of the service user management information reference unit 334.

If the service user management information reference unit 334 receives a request to refer to the service user attribute information from the mobile terminal 2 via the server communication unit 31 (step S400 - Yes), the service user management information reference unit 334 creates the attribute information display screen display data (step S402). Then, the service user management information reference unit 334 proceeds to the transmission processing of the attribute information display screen display data or the like (step S410).

On the other hand, if the service user management information reference unit 334 receives a request to refer to the service action work procedure (step S404 - Yes), the service user management information reference unit 334 creates the work procedure display screen display data (step S406). Then, the service user management information reference unit 334 proceeds to the transmission processing of the attribute information display screen display data or the like (step S410).

On the other hand, if the service user management information reference unit 334 receives a request to refer to the service provision record (step S404 - No), the service user management information reference unit 334 creates the service provision record display screen display data (step S408).

The service user management information reference unit 334 transmits the created attribute information display screen display data or the like to the mobile terminal 2 via the server communication unit 31 (step S410).

FIG. 10B is a diagram illustrating an example of an operation flow of the service user management information updating unit 335.

If the service user management information updating unit 335 receives a request to transmit the service provision record input form from the mobile terminal 2 via the server communication unit 31, the service user management information updating unit 335 creates the service provision record input screen display data (step S500).

The service user management information updating unit 335 transmits the created service provision record input screen display data to the mobile terminal 2 via the server communication unit 31 (step S502).

If the service user management information updating unit 335 receives a request to store the service provision record from the mobile terminal 2 via the server communication unit 31, the service user management information updating unit 335 stores the service provision record in the server storage unit 32 (step S504). Then, the service user management information updating unit 335 terminates the processing.

As explained above, in scheduling of the service provider, by preferentially assigning the part-time staff over the full-time staff, it is made easier for the part-time staff to perform the service provision task in the time period desired by him/herself, and therefore the chance to work of the service provider and to improve the chance to use a service of the service user may be improved.

The present invention is not limited to the present embodiment. For example, in the present embodiment, although the part-time staffs each desired date and time of service provision is assigned to the service user's each desired date and time of service use, and the full-time staffs each desired date and time of service provision is assigned to the service user's each unassigned date and time of service use, the full-time staffs each desired date and time of service provision and the part-time staffs each desired date and time of service provision to the service user's each desired date and time of service use may be simultaneously assigned, in accordance with a restriction condition that gives priority to the part-time staff's each desired date and time of service provision over the full-time staffs each desired date and time of service provision. Thus, the same working and effect as those of the present embodiment may be obtained.

In the present embodiment, although the assignment processing is performed for all the service users and the service providers, the assignment processing for part of the service users and the service providers may be performed. For example, the assignment processing for the service users belonging to a predetermined group and for the service providers belonging to the same predetermined group may be performed, after grouping the service providers based on attributes (e.g., qualification or the like) or based on the relationship (e.g., chemistry or the like) with service users belonging to the assignment-target group as well as also grouping the service users based attributes (e.g., address or the like). Further, in accordance with the results of grouping, the assignment-target service user group and/or service provider group may be sequentially change or enlarge. Then, the burden of assignment processing may be reduced.

In the present embodiment, although the assignment processing is performed based on the service user's desired date and time of service use and the service provider's desired date and time of service provision, the assignment processing may be performed based on conditions other than the date and time (e.g., the address of the service user and the area where the service provision by the service provider is available, the service action to be used by the service user and the service action that the service provider can provide, the service user's desired service provider and the service provider's desired service user, etc.). Then, preferable service scheduling may be crated.

Further, it is possible to provide the computer programs for causing a computer to implement each function included in the terminal processing unit 25 and the server processing unit 33 in the form of being stored in a storage medium that can be read by the computer, such as a semiconductor storage medium, a magnetic storage medium, and an optical storage medium, and to install the computer programs into the terminal storage unit 22 and/or the server storage unit 32 from the storage medium by using publicly known setup programs or the like.

It should be noted that the person skilled in the art can perform a variety of alterations, replacements, and modifications without departing from the sprit and scope of the present invention.

### Explanations of letters or numerals

- 1: visiting service management support system
- 2: mobile terminal
- 21: terminal communication unit
- 22: terminal storage unit
- 23: operation unit
- 24: display unit
- 25: terminal processing unit
- 251: browsing execution unit
- 3: server
- 31: server communication unit
- 32: server storage unit
- 33: server processing unit
- 331: service scheduling information creation unit
- 332: service scheduling information reference unit
- 333: service scheduling information updating unit
- 334: service user management information reference unit
- 335: service user management information updating unit
- 4: base station
- 5: mobile communication network
- 6: gateway
- 7: Internet

## Claims

1. A server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server comprising:
a communication unit configured to communicate with the first terminal and the second terminal, respectively;
a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use;
a service scheduling information creation unit configured to assign each date and time indicated by the second service provider's desired date and time information to each date and time indicated by the service user's desired date and time information, to further assign each date and time indicated by the first service provider's desired date and time information to each date and time to which each date and time indicated by the second service provider's desired date and time information is not assigned among each date and time indicated by the service user's desired date and time information, to create service scheduling information including a service user, a first service provider or a second service provider, and a service provision date and time based on the assignment results, and to store the service scheduling information in the storage unit; and
a service scheduling information reference unit configured to transmit the service scheduling information to the first terminal or the second terminal via the communication unit.

2. A server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server comprising:
a communication unit configured to communicate with the first terminal and the second terminal, respectively;
a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use;
a service scheduling information creation unit configured to simultaneously assign each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information, to create service scheduling information including a service user, a first service provider or a second service provider, and a service provision date and time based on the assignment results, and to store the service scheduling information in the storage unit; and
a service scheduling information reference unit configured to transmit the service scheduling information to the first terminal or the second terminal via the communication unit.

3. The server according to claim 1 or 2, wherein
if the assignment results are modified, the service scheduling information creation unit performs the assignment processing again so that the modification is reflected in the assignment results.

4. The server according to any one of claims 1 to 3, wherein
the service scheduling information creation unit performs the assignment processing based on the service user's desired date and time information relating to a service user belonging to a predetermined group of a plurality of groups, and the first service provider's desired date and time information relating to a first service provider belonging to a predetermined group of a plurality of groups having a system different from that of the plurality of groups and the second service provider's desired date and time information relating to a second service provider.

5. The server according to any one of claims 1 to 4, wherein
the storage unit further stores service user management information including service user attribute information and a service provision record relating to the service user for each service user, and
the server further comprises a service user management information reference unit configured to transmit the service user management information to the first terminal or the second terminal via the communication unit.

6. The server according to claim 5, further comprising a service user management information updating unit configured to transmit a service provision record input form relating to a service user to the first terminal or the second terminal via the communication unit and to store the service provision record received from the first terminal or the second terminal via the communication unit in the storage unit as a service provision record relating to the service user.

7. A control method of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control method comprising, by the server:
assigning each date and time indicated by the second service provider's desired date and time information to each date and time indicated by the service user's desired date and time information;
assigning each date and time indicated by the first service provider's desired date and time information to each date and time to which each date and time indicated by the second service provider's desired date and time information is not assigned among each date and time indicated by the service user's desired date and time information;
creating service scheduling information including a service user, a first service provider or a second service provider, and a service provision date and time based on the assignment results;
storing the service scheduling information in the storage unit; and
transmitting the service scheduling information to the first terminal or the second terminal.

8. A control method of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control method comprising, by the server:
simultaneously assigning each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information;
creating service scheduling information including a service user, a first service provider or a second service provider, and a service provision date and time based on the assignment results;
storing the service scheduling information in the storage unit; and
transmitting the service scheduling information to the first terminal or the second terminal.

9. A control program of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control program causing the server to;
assign each date and time indicated by the second service provider's desired date and time information to each date and time indicated by the service user's desired date and time information;
assign each date and time indicated by the first service provider's desired date and time information to each date and time to which each date and time indicated by the second service provider's desired date and time information is not assigned among each date and time indicated by the service user's desired date and time information;
create service scheduling information including a service user, a first service provider or a second service provider, and a service provision date and time based on the assignment results;
store the service scheduling information in the storage unit; and
transmit the service scheduling information to the first terminal or the second terminal.

10. A control program of a server in a visiting service management support system including a first terminal relating to a first service provider who provides a visiting service in a first type working pattern in which the first service provider works for a long time and continuously, a second terminal relating to a second service provider who provides a visiting service in a second type working pattern that is different from the first type working pattern and in which the second service provider works for a shorter time and less continuously than the first type working pattern, and a server capable of communicating with the first terminal and the second terminal, respectively, the server including a storage unit configured to store first service provider's desired date and time information indicating a first service provider's desired date and time of service provision, second service provider's desired date and time information indicating a second service provider's desired date and time of service provision, and service user's desired date and time information indicating a service user's desired date and time of service use, the control program causing the server to:
simultaneously assign each date and time indicated by the first service provider's desired date and time information and each date and time indicated by the second service provider's desired date and time information to each date and time indicated by the service user's desired date and time information in accordance with a restriction condition that gives priority to each date and time indicated by the second service provider's desired date and time information over each date and time indicated by the first service provider's desired date and time information;
create service scheduling information including a service user, a first service provider or a second service provider, and a service provision date and time based on the assignment results;
store the service scheduling information in the storage unit; and
transmit the service scheduling information to the first terminal or the second terminal.
